# EUROPEAN PATENT APPLICATION

(11) **EP 3 020 416 A1**
(43) Date of publication of application: **18.05.2016**
(21) Application number: 14822159.1
(22) Date of filing: 04.07.2014
(51) Int. Cl.: A61K 47/38, A61K 9/46

(54) **ULTRAFAST-DISINTEGRATING TABLET AND METHOD FOR MANUFACTURING SAME**

(30) Priority: 06.07.2013 JP 2013142189; 12.12.2013 JP 2013257205
(71) Applicant: Daicel Corporation, Osaka-shi, Osaka 530-0011 (JP)
(72) Inventor: HIRAMURA, Takahiro, Tokyo 108-8230 (JP); ITAYA, Sae, Himeji-shi Hyogo 671-1281 (JP); HASHIKAWA, Naohiro, Himeji-shi Hyogo 671-1281 (JP); OKABAYASHI, Tomohito, Himeji-shi Hyogo 671-1281 (JP); SAKAGUCHI, Anan, Himeji-shi Hyogo 671-1281 (JP)
(74) Representative: Power, David
(86) International application number: PCT/JP2014/067936
(87) International publication number: WO 2015/005241

(57) **Abstract**

An object of the present invention is to provide an orally disintegrating tablet (ultrafast-disintegrating tablet) having an extremely high disintegrability (short disintegration time), and a high tablet hardness, and to provide a simple method for the production of said ultrafast-disintegrating tablet without such a complicated process as freeze-drying.

This invention relates to an orally disintegrating tablet comprising a first disintegrator component of an acid-type carboxymethylcellulose, and having a tablet hardness of 10 N or more and a disintegration time in water of less than 15 seconds, a method for the production of said orally disintegrating tablet, and to a disintegrative particulate composition for use in said method.

## Description

### Technical Field

The present invention relates to an orally disintegrating tablet having an extremely short oral disintegration time and an extremely high tablet hardness, and to a method for producing thereof.

### Background Art

In the past, orally disintegrating tablets have been developed as highly convenient forms which can safely be taken by patients who have difficulty in swallowing drugs, elderly people, children, etc., and which can easily be taken without water. It is important that the orally disintegrating tablets have sufficient breaking strength (tablet hardness) such that any cracks, powdering, etc. are not caused in the tablets during production or transportation of the tablets or during breaking their seals in the same manner as general tablets, and also, it is important that the orally disintegrating tablets have excellent disintegrability (disintegration time) such that the tablets immediately disintegrate in the oral cavity.

The tablet hardness and disintegrability are mutually opposing properties. In general, when a molding pressure is increased to increase the hardness, the disintegration time will tend to be prolonged, and, when the molding pressure is reduced to shorten the disintegration time, the hardness will tend to be smaller. Therefore, various technologies have been developed in order to cope with both the two properties or to achieve an optimal balance between the two properties.

Furthermore, the components of particles, granulation methods, etc. have been studied in order to impart superior moldability to the particles or particulate compositions constituting tablets.

It is well known that although the orally disintegrating tablets have improved medicine-taking compliance by patients, some patients having tendency to strongly reject the taking of medicine would vomit the tablets having the oral disintegration time in a range of bout 20-30 seconds. Accordingly, if a tablet has an extremely high disintegrability with the disintegration time of a few seconds, it can be easily administered to said patients since it will be disintegrated before they may feel uncomfortable when they take it.

Zydis (Registered Trademark) is known as a technique for the production of such tablet having an extremely high oral disintegrability, that is, an "ultrafast-disintegrating tablet." This technique has been developed by Cardinal Health Co. (Catalent Pharma Solutions, LLC) for the production of an oral solid formulation. As shown in PTL 1, it comprises preparing suspension of bulk (medicinal ingredient) and mannitol using gelatin as a supporting material and filling the suspension into a blister, followed by freeze-drying to give a rapidly dispersing solid formulation for an oral administration.

Furthermore, PTL 2 discloses an invention relating to a method for the production of a multi-phasic, lyophilized, fast-dissolving dosage form. It is prepared by sequential dosing of a formulation containing a forming agent of non-gelling matrix and a formulation containing a forming agent of gelling gelatin, followed by freeze-drying to give a multi-phasic, lyophilized, fast-dissolving dosage form (FDDF) for the delivery of a pharmaceutically active ingredient. For example, non-gelling gelatin and gelling gelatin are used for the forming agents of non-gelling matrix and gelling matrix, respectively.

### Related Arts

### Patent Literature

PTL 1: Specification of Japanese Patent No. 4943581
PTL 2: JP-A-2013-522308

### Summary of Invention

### Problems to be solved by the Invention

However, the above-mentioned conventional techniques would require a specialized device for freeze-drying, and could not use a tablet machine for the production of an usual tablet with a high production efficiency. And, the above ultrafast-disintegrating tablet produced by the conventional techniques has an extremely low tablet hardness. It has been therefore desired an ultra-fast disintegrating tablet showing such a level of tablet hardness as allowing for a usual PTP package.

Accordingly, an object of the present invention is to solve such technical problems as found in the conventional ultrafast-disintegrating tablet, and thus, is to provide an orally disintegrating tablet (ultrafast-disintegrating tablet) having such an extremely high disintegrability (short disintegration time) that a patient's taking of medicine can be certainly confirmed, and such a high tablet hardness that cracking and lacking of the tablet is expected to be reduced to a practical level. A further object of the present invention is to provide a simple method for the production of said ultrafast-disintegrating tablet without a complicated process such as freeze-drying.

### Means to Solve the Problem

The present inventors found that an ultrafast-disintegrating tablet having an extremely short disintegration time and a high tablet hardness can be obtained in the conventional granulation step, by using an acid-type carboxymethylcellulose as one of disintegrator components of the orally disintegrating tablet, leading to completion of this invention.

More specifically, the present invention is to provide the following aspects.

### [Aspect 1]

An orally disintegrating tablet comprising a first disintegrator component of an acid-type carboxymethylcellulose, and having a tablet hardness of 10 N or more and a disintegration time in water of less than 15 seconds.

### [Aspect 2]

The orally disintegrating tablet according to Aspect 1, further comprising crystalline cellulose.

### [Aspect 3]

The orally disintegrating tablet according to Aspect 1 or 2, further comprising bicarbonate.

### [Aspect 4]

The orally disintegrating tablet according to any one of Aspects 1 to 3, wherein a specific surface area of the tablet is of from 0.75 to 1.50 mm²/mg.

### [Aspect 5]

A method for the production of the orally disintegrating tablet according to any one of Aspects 1 to 4, comprising mixing a disintegrative particulate composition with a medicinal ingredient, and subjecting the resulting mixture to tableting.

### [Aspect 6]

A method for the production of the orally disintegrating tablet according to Aspect 5, comprising a wet granulation step in the production of the disintegrative particulate composition.

### [Aspect 7]

A method for the production of the orally disintegrating tablet according to Aspect 5 or 6, comprising a two-stage granulation step in the production of the disintegrative particulate composition.

### [Aspect 8]

A method for the production of the orally disintegrating tablet according to any one of Aspects 5 to 7, wherein the tableting is carried out at a tablet compression force of from 1 to 30 kN.

### [Aspect 9]

A method for the production of the orally disintegrating tablet according to Aspect 8, wherein the tableting is carried out at a tablet compression force of from 2 to 30 kN.

### [Aspect 10]

A disintegrative particulate composition comprising a first disintegrator component of an acid-type carboxymethylcellulose, for use in the method for the production of the orally disintegrating tablet according to any one of Aspects 5 to 9.

### Advantageous Effects of Invention

According to the present invention, the ultrafast-disintegrating tablet having the extremely short oral disintegration time and the high tablet hardness can be easily produced by means of such a device as used for the conventional tablets.

### Concise Explanation of Drawings

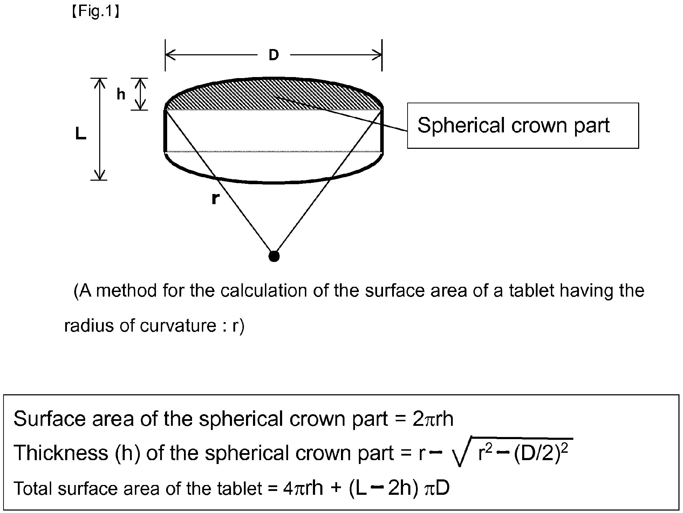

Fig. 1 shows a method for the calculation of the surface area of the orally disintegrating tablet according to the present invention.

### Description of Embodiments

The present invention relates to the orally disintegrating tablet comprising the first disintegrator component of the acid-type carboxymethylcellulose, and having the tablet hardness of 10 N or more and the disintegration time in water of less than 15 seconds.

It is preferable for the orally disintegrating tablet to further comprise bicarbonate in order to further improve the above properties. An amount of the bicarbonate may be optionally determined by those skilled in the art depending on a kind and amount of a medicinal ingredient and other components in the disintegrative particulate composition and the like. It is usually from 0.1 to 20 % by weight, preferably from 0.5 to 15 % by weight, and more preferably from 0.5 to 10% by weight of the orally disintegrating tablet. The bicarbonate used in the present invention may be any compounds known in the art, which will produce carbon dioxide and is pharmaceutically acceptable, including, for example, sodium bicarbonate, potassium bicarbonate, lithium bicarbonate, rubidium bicarbonate and cesium bicarbonate. It is not necessary to include gelatin in the orally disintegrating tablet according to the present invention, which is substantially essential as a supporting material in the prior ultrafast-disintegrating tablet.

It is desired to make the time for the progress of disintegration to such an extent that patients could not vomit the tablet administered any more, so that the patients won't feel uncomfortable and their vomit of the tablet will be inhibited. It is also desired to complete the disintegration and administration of the tablet as soon as possible so that the following administrations won't be rejected by the patient. In order to realize such excellent administration, the disintegration time in water of the tablet is preferably less than 10 seconds, more preferably less than 7 seconds, and most preferably less than 5 seconds. From a view point of a production and use of the tablet, a relatively high tablet hardness is required, being preferably 20 N or more and more preferably 30 N or more.

Values of these physical properties were measured based on the following conditions/methods.

Hardness: a hardness (N) was measured with a Kiya hardness tester (KHT-40N, Fujiwara Scientific Company Co., Ltd.).

Disintegration time in water: a disintegration time in water was measured with a disintegration tester (NT-4HF, TOYAMA SANGYO CO., LTD.) in accordance with the method described in the Japanese Pharmacopoeia provided that an auxiliary disk was not used. However, the auxiliary disk was used for the tablets comprising bicarbonates.

The measurements for the hardness and disintegration time were each repeated four to six times, and average values thereof were regarded as measurement results.

The disintegration time in water measured above is in correlation with the time for the progress of disintegration to such an extent that the patients could not vomit the tablet administered any more, and with the oral disintegration time (time for completion of the disintegration). The oral disintegration time was measured in Examples as follows: one tablet was taken in the oral cavity, and, while keeping a state in which the tablet was placed between the tongue and the upper jaw without applying any pressure thereto, the time required for the tablet to be completely disintegrated was measured. The measurements were each repeated three times by a few adults of both sexes, and average values thereof were regarded as measurement results.

In order to obtain the above properties, it is desirable that a specific surface area of the tablet is of from 0.75 to 1.50 mm²/mg, preferably of from 0.90 to 1.50 mm²/mg, and more preferably of from 0.90 to 1.30 mm²/mg.

The medicinal ingredient comprised in the orally disintegrating tablet according to the present invention includes a pharmaceutical ingredient, and nutrient ingredient comprised in foods and health food products. The medicinal ingredient may be added as such or in a coated or granulated form for the purpose of sustained release or masking of bitterness of the medicinal ingredient.

There is no limitation on a application or kind of the medicinal ingredients comprised in the orally disintegrating tablet according to the present invention, which may include, for example, agents affecting each organ such as the central nervous system, peripheral nervous system, a sensory organ, a circulatory organ, a respiratory organ and a digestive organ and an urogenital organ; hormone drug; agents affecting metabolism such as a vitamin drug, an analeptic, an agent affecting blood and body fluid; agents affecting the function of tissue and cell such as an agent activating cellular function, an agent affecting tumors, an radioactive medicine, an anti-allergic agent; medicines based on a medical prescription relating to herbal medicines and Chinese medicines; antibiotics; agents for chemotherapy, biological drug; agents for pathogenic organisms such as parasites; agents for dispersing use, diagnosis, public health and external diagnosis.

The orally disintegrating tablet according to the present invention may optionally include other pharmaceutically-acceptable components such as excipients, surfactants, lubricants, acidulants, sweeteners, corrigents, flavoring agents, colorants, and stabilizing agents, when needed. As these optional components, for example, appropriate ingredients described in "Japanese Pharmaceutical Excipients Directory" (YAKUJI NIPPO LIMITED) or the Japanese Pharmacopoeia can be used. Also, the blending ratios of each ingredient (component) are not particularly limited as long as the expected effects of the present invention are brought about, and the blending ratios can properly be determined by those skilled in the art. Furthermore, the tablet can comprise various disintegrators comprised in the following disintegrative particulate composition. The orally disintegrating tablet can be formulated by any methods known to those skilled in the art, for example, by tableting.

One of the preferable methods for the production of the orally disintegrating tablet comprises mixing the disintegrative particulate composition with the medicinal ingredient (or a pharmaceutical composition containing the medicinal ingredient) and other optional components, and subjecting the resulting mixture to tableting by means of any suitable tableting machine known in the art at a tablet compression force of from 1 to 30 kN, preferably of from 2 to 30 kN, more preferably of from 2 to 15 kN, and most preferably of from 3 to 15 kN. Alternatively, an "externally lubricating tableting method" may be adopted, wherein a lubricant such as magnesium stearate is sprayed or applied in advance on a mortar and pestle of the tableting machine. The present invention therefore concerns the disintegrative particulate composition for use in the above method as well.

There is no limitation on the conditions (states) of the medicinal ingredients and the like, being, for example, a powdered form. Mixing (solid trituration) of the disintegrative particulate composition with the medicinal ingredient and tableting may be carried out by any method or means known to those skilled in the art. An amount of the active ingredients in the orally disintegrating tablet may be controlled to a suitable administration dose during the above steps depending on a subject, purpose and the like for the administration.

Four mechanisms of "wicking", "swelling", "deformation" and "repulsion" have been proposed as mechanisms of disintegration of tablets or the like. Among them, "wicking" is a disintegration mechanism which proceeds upon weakened binding force between particles included in the tablet as a result of water permeation through components such as disintegrators included in the tablet. As a typical example of a disintegrator having a higher effect to promote such "wicking", an acid-type carboxymethylcellulose has been known. Also, "swelling" is a disintegration mechanism which proceeds upon swelling of disintegrators themselves as a result of water permeation through the disintegrators.

The acid-type carboxymethylcellulose, which is the first disintegrator component included in the disintegrative particulate composition of the present invention, is a substance called carmellose, and has been used as a pharmaceutical additive. In the same manner as the acid-type carboxymethylcellulose, for example, both a calcium salt of carboxymethylcellulose and a cross-linked product of carboxymethylcellulose sodium are water-insoluble, and have been used as disintegrator for tablets, etc. On the other hand, a sodium salt of carboxymethylcellulose is water-soluble, and has been used for purposes of a binder, etc. In addition, in some cases, a salt of carboxymethylcellulose may be referred to as carmellose.

The disintegrative particulate composition further comprises crystalline cellulose known to those skilled in the art. As typical examples of such crystalline cellulose, commercially-available products such as "Avicel" (FMC Corporation), "CEOLUS" (Asahi Kasei Chemicals Corp.), and "VIVAPUR" (RETTENMAIER) can be mentioned.

For the second disintegrator component of the disintegrative particulate composition of the present invention, any disintegrators other than the acid-type carboxymethylcellulose which have been known to those skilled in the art can be used. However, in order to obtain combined effects of the different disintegration mechanisms as shown above, it is preferable that a disintegrator having a superior effect to promote a mechanism other than "wicking" (e.g. "swelling") be used as the second disintegrator component. Suitable examples of such a disintegrator include crospovidone, sodium croscarmellose, sodium carboxymethyl starch, low substituted hydroxypropylcellulose, calcium carboxymethylcellulose, hydroxypropyl starch, and starch. Additionally, "crospovidone" is a common name for a cross-linked polymer of 1-vinyl-2-pyrrolidone, and "sodium croscarmellose" is a common name for a cross-linked product of sodium carboxymethylcellulose.

Among them, one, or any combination of two or more components selected from crospovidone, sodium croscarmellose, sodium carboxymethyl starch, low substituted hydroxypropylcellulose and calcium carboxymethylcellulose is preferable.

The disintegrative particulate composition further comprises an excipient. Typical examples of the excipient are sugars or sugar alcohols such as mannitol, erythritol, sorbitol, D-glucitol (maltitol), xylitol, trehalose, lactose, maltose, and. Moreover, as preferable examples thereof, mannitol erythritol, trehalose, sorbitol and D-glucitol (maltitol) can be mentioned. As the excipient, two or more types of compounds properly selected from these compounds may also be used.

The disintegrative particulate composition preferably comprises crystalline cellulose in order to further improve the properties of the disintegrating tablet according to the present invention. Furthermore, various types of optional components known to those skilled in the art may properly be added and mixed into the disintegrative particulate composition of the present invention, for the purpose of adjusting various characteristics such as the disintegrating force, binding force and ease in taking the tablet. As examples of such components, fluidizing agents, sweetening agents, flavoring agents and coloring agents can be mentioned.

The amount of each component blended in the disintegrative particulate composition of the present invention can properly be determined by those skilled in the art, depending on, for example, a type of the component, a type and purpose of the medicinal ingredient for which the disintegrative particulate composition is to be used, or a purpose of the final product, i.e. the orally disintegrating tablet. In general, relative to a total weight of the disintegrative particulate composition, the amount of the first disintegrator component is within a range of 10% to 50% by weight, the amount of the second disintegrator component is within a range of 1% to 20% by weight, the amount of the excipient is within a range of 30 to 88% by weight, and the amount of the crystalline cellulose is within a range of 1% to 40% by weight.

The disintegrative particulate composition according to the present invention may be produced by any method known to those skilled in the art. For example, it may be produced by a two-stage granulation step comprising a first wet granulation step using any one or two of the three components and a second wet granulation step using at least the granules obtained in the first wet granulation step and the remaining component(s) not used in the first wet granulation step, or a three-stage granulation step further comprising a third step of mixing other components with the granules obtained in the second wet granulation step.

Furthermore, the disintegrative particulate composition according to the present invention may be produced by one wet granulation step using all of the components together.

In each method of the above production method, each granulation step is carried out by a method in which each component is dispersed in the presence of water, and the dispersion is dried to form complexes, i.e. by a wet granulation process. As specific examples of a wet granulation process, spray methods such as spray drying, tumbling granulation, agitation granulation and fluidized-bed granulation; the freeze-drying method; kneading granulation, and the like can be mentioned. The composition can be produced by any of these methods known to those skilled in the art.

Since disintegrators such as an acid-type carboxymethylcellulose are hydrophilic, by carrying out a manipulation of applying a physical force such as by agitation or the like in the presence of water according to the wet granulation, the aggregated state in the dry powder will convert into a state in which particles are more dispersed. Dispersion can most easily be carried out by the fluidized-bed granulation process in which dispersion by water spraying and drying are carried out, spray drying, tumbling granulation, agitation granulation, etc., and also, drying speeds in these methods are high. Therefore, these methods are preferable.

Among them, the fluidized-bed granulation process is a granulation method in which water, an aqueous solution including a binder, or the like is sprayed onto powder, while blowing the powder up by hot air, and adjustment of spraying conditions, etc. is easy in this method. Therefore, the fluidized-bed granulation process is the most preferable method.

Furthermore, tose skilled in the art can properly determine various conditions in each granulation step, such as the spraying speed, the supply air temperature, the exhaust temperature, and the air supply rate, depending on types or amounts of the components, etc.

In each granulation step, as a medium for the spray liquid, a solvent acceptable in pharmaceuticals or foods, such as water, ethanol, methanol or acetone, can be mentioned. Alternatively, as the spray liquid, for example, an aqueous solution in which less than 10% of the component (s) for the disintegrative particulate composition is dissolved can be mentioned, and, in particular, water or such an aqueous solution is preferable.

It is preferable that the disintegrative particulate composition of the present invention have the following physical properties:
(1) an average particle size of 50 to 200 microns; and
(2) a water content of 0.5% to 6% by weight.

In addition, these physical properties are measured by using the following methods and conditions.

The average particle size: 2 g of the disintegrative particulate composition is subjected to a measurement with a φ75 mm automatic shaking sieve device (Type "M-2", Tsutsui Scientific Instruments Co., Ltd.).

The water content: 5 g of the disintegrative particulate composition is subjected to a measurement using a halogen water content measuring device (Type "HB43", METTLER TOLEDO K.K.).

In addition, contents of all related art documents cited in the present specification are incorporated herein by reference.

Hereinafter, the present invention will more specifically be described with reference to Examples. However, the present invention is not considered to be limited to the Examples.

### Examples

### (Production of the disintegrative particulate composition)

In the first wet granulation step, 280 g of mannitol (D-mannitol, Merck Ltd.), 75 g of carmellose (NS-300, GOTOKU CHEMICAL CO., LTD.) and 100 g of a crystalline cellulose (CEOLUS PH-101, Asahi Kasei Chemicals Corp.) were charged to a fluidized-bed granulator (LAB-1, Powrex Corporation), and 240 g of purified water was sprayed onto the resulting mixture at a rate of 24 g/minute to thereby obtain granules. In the second wet granulation step, 40 g of crospovidone (Polyplasdone INF-10, ISP Japan) was added to the granules and 300 g of purified water was sprayed onto the resulting mixture at a rate of 10 g/minute to thereby obtain granules (a disintegrative particulate composition of the present invention). The resulting granules had the following values for physical properties: (1) an average particle size of 93 microns and (2) a water content of 2.3% by weight.

### (Production of the orally disintegrating tablet)

The thus obtained granules were then subjected to tableting at a tablet compression force of from 3 kN to 6 kN with a simple tableting machine (HANDTAB-100, ICHIHASHI-SEIKI Co., Ltd.) to thereby obtain angled-corner flat tablets having a diameter of 0.8 mm and a weight of from 100 to 200 mg (Examples 1-6).

0.5 parts by weight of magnesium stearate (Taihei Chemical Industrial Co. Ltd.) was added to 99.5 parts by weight of the resulting granules, and these were mixed. The mixture was then subjected to tableting at tablet compression forces of 6 kN and 8 kN with the simple tableting machine (HANDTAB-100, ICHIHASHI-SEIKI Co., Ltd.) to thereby obtain tablets having a diameter of 8.0 mm, R6.5, and a weight of 150 mg (Examples 7 and 8).

0.5 parts by weight of magnesium stearate (Taihei Chemical Industrial Co. Ltd.) was added to 99.5 parts by weight of the resulting granules, and these were mixed. The mixture was then subjected to tableting at a tablet compression force of 5 kN with a rotating tableting machine (VIRGO, KUKUSUI SEISAKUSHO LTD.) to thereby obtain an angled-corner scored flat tablet having a diameter of 8.0 mm and a weight of 150 mg (Example 9).

0.5 parts by weight of magnesium stearate (Taihei Chemical Industrial Co. Ltd.) and 0.3 parts by weight of sucralose (San-Ei Gen F.F.I.,Inc.) were added to 99.2 parts by weight of the resulting granules, and these were mixed. The mixture was then subjected to tableting at tablet compression force of 6 kN with a rotating tableting machine (REGASUS, KUKUSUI SEISAKUSHO LTD.) to thereby obtain an angled-corner scored flat tablet having a diameter of 8.0 mm and a weight of 150 mg (Example 10).

The resulting granules was then subjected to tableting at a tablet compression force of 4 kN with the simple tableting machine (HANDTAB-100, ICHIHASHI-SEIKI Co., Ltd.) wherein magnesium stearate (Taihei Chemical Industrial Co. Ltd.) had been applied in advance on the mortar and pestle of the tableting machine to thereby obtain an angled-corner flat tablet having a diameter of 8.0 mm and a weight of 100 mg (Example 11).

3 parts by weight of sodium bicarbonate (Wako Pure Chemical Industries, Ltd.), 0.5 parts by weight of magnesium stearate (Taihei Chemical Industrial Co. Ltd.) and 0.5 parts by weight of sucralose (San-Ei Gen F.F.I.,Inc.) were added to 96.0 parts by weight of the resulting granules, and these were mixed. The mixture was then subjected to tableting at a tablet compression force of 2 kN with the simple tableting machine (HANDTAB-100, ICHIHASHI-SEIKI Co., Ltd.) to thereby obtain an angled-corner flat tablet having a diameter of 6.0 mm and a weight of 90 mg (Example 12).

3 parts by weight of sodium bicarbonate (Wako Pure Chemical Industries, Ltd.), 0.5 parts by weight of magnesium stearate (Taihei Chemical Industrial Co. Ltd.) and 0.5 parts by weight of sucralose (San-Ei Gen F.F.I.,Inc.) were added to 96.0 parts by weight of the resulting granules, and these were mixed. The mixture was then subjected to tableting at a tablet compression force of 4 kN with a rotating tableting machine (HT-AP18SS-II, HATA TEKKOSHO CO.,LTD.) to thereby obtain an angled-corner scored flat tablet having a diameter of 7.0 mm and a weight of 100 mg (Example 13).

5 parts by weight of sodium bicarbonate (Wako Pure Chemical Industries, Ltd.), 0.5 parts by weight of magnesium stearate (Taihei Chemical Industrial Co. Ltd.) and 0.5 parts by weight of sucralose (San-Ei Gen F.F.I.,Inc.) were added to 94.0 parts by weight of the resulting granules, and these were mixed. The mixture was then subjected to tableting at a tablet compression force of 2 kN with the simple tableting machine (HANDTAB-100, ICHIHASHI-SEIKI Co., Ltd.) to thereby obtain an angled-corner flat tablet having a diameter of 6.0 mm and a weight of 90 mg (Example 14).

3 parts by weight of sodium bicarbonate (Wako Pure Chemical Industries, Ltd.) and 0.5 parts by weight of sucralose (San-Ei Gen F.F.I.,Inc.) were added to 96.5 parts by weight of the resulting granules, and these were mixed. The mixture was then subjected to tableting at a tablet compression force of 2 kN with the simple tableting machine (HANDTAB-100, ICHIHASHI-SEIKI Co., Ltd.) wherein magnesium stearate (Taihei Chemical Industrial Co. Ltd.) had been applied in advance on the mortar and pestle of the tableting machine simple tableting machine (HANDTAB-100, ICHIHASHI-SEIKI Co., Ltd.) to thereby obtain an angled-corner flat tablet having a diameter of 6.0 mm and a weight of 90 mg (Example 15).

8 parts by weight of crospovidone (Polyplasdone INF-10, ISP Japan) and 0.5 parts by weight of magnesium stearate (Taihei Chemical Industrial Co. Ltd.) were added to 91.5 parts by weight of the resulting granules, and these were mixed. The mixture was then subjected to tableting at a tablet compression force of 2 kN with the simple tableting machine (HANDTAB-100, ICHIHASHI-SEIKI Co., Ltd.) to thereby obtain an angled-corner flat tablet having a diameter of 6.0 mm and a weight of 90 mg (Example 16).

### [Comparative Examples]

0.5 parts by weight of magnesium stearate (Taihei Chemical Industrial Co. Ltd.) was added to 99.5 parts by weight of the resulting granules, and these were mixed. The mixture was then subjected to tableting at tablet compression forces of 6 kN and 8 kN with the simple tableting machine (HANDTAB-100, ICHIHASHI-SEIKI Co., Ltd.) to thereby obtain tablets having a diameter of 8.0 mm, R6.5 and a weight of 250 mg (Comparative Examples 1 and 2).

### [Various Properties of tablets]

Various properties of the tablets according to the Examples and Comparative Examples are shown in Table 1 below. The data in Table 1 show that the orally disintegrating tablet according to the present invention is an ultrafast-disintegrating tablet having such an extremely high disintegrability as a disintegration time in water of less than 15 seconds, and such a high tablet hardness as of 10 N or more.

**[Table 1]**

| | Example 1 | Example 2 | Example 3 | Example 4 | Example 5 | Example 6 |
|---|---|---|---|---|---|---|
| Tablet Weight (mg) | **100** | **150** | **150** | **150** | **200** | **200** |
| Specific Surface Area (mm²/mg) | **1.49** | **1. 17** | **1. 15** | **1. 11** | **0. 97** | **0.94** |
| Tablet compression Force (kN) | **6** | **3** | **4** | **6** | **3** | **4** |
| Tablet Hardness (N) | **34** | **23** | **33** | **52** | **31** | **42** |
| Disintegration time in water (seconds) | **6** | **7** | **9** | **9** | **9** | **10** |
| Oral Disintegration time (seconds) | **6** | **7** | **7** | **6** | **8** | **8** |

**[Table 2]**

| | Example 7 | Example 8 | Example 9 | Example10 | Example11 | Example12 |
|---|---|---|---|---|---|---|
| Tablet Weight (mg) | **150** | **150** | **150** | **150** | **100** | **90** |
| Specific Surface Area (mm²/mg) | **0.92** | **0.90** | **1.13** | **1.12** | **1.46** | **1.22** |
| Tablet compression Force (kN) | **6** | **8** | **5** | **6** | **4** | **2** |
| Tablet Hardness (N) | **35** | **45** | **22** | **32** | **18** | **15** |
| Disintegration time in water (seconds) | **11** | **12** | **10** | **9** | **5** | **4** |
| Oral Disintegration time (seconds) | **8** | **8** | **8** | **10** | **5** | **6** |

**[Table 3]**

| | Example 13 | Example 14 | Example 15 | Example 16 | Comparative Example 1 | Comparative Example 2 |
|---|---|---|---|---|---|---|
| Tablet Weight (mg) | **100** | **90** | **90** | **90** | **250** | **250** |
| Specific Surface Area (mm²/mg) | **1.26** | **1.21** | **1.22** | **1.25** | **0.70** | **0.69** |
| Tablet compression Force (kN) | **4** | **2** | **2** | **2** | **6** | **8** |
| Tablet Hardness (N) | **16** | **18** | **23** | **24** | **93** | **119** |
| Disintegration time in water (seconds) | **6** | **4** | **7** | **7** | **17** | **21** |
| Oral Disintegration time (seconds) | **5** | **6** | **6** | **6** | **15** | **16** |

### Industrial Applicability

The present invention has enabled to provide an orally disintegrating tablet (ultrafast-disintegrating tablet) having not only advantages that it can safely be taken by patients who have difficulty in swallowing drugs, elderly people, children, etc. and can easily be taken without water, but also further advantages that it has such an extremely high disintegrability (short disintegration time) that the taking of medicine by patients having tendency to strongly reject it can be certainly confirmed, and such a high tablet hardness that cracking and lacking of the tablet is expected to be reduced to a practical level.

## Claims

1. An orally disintegrating tablet comprising a first disintegrator component of an acid-type carboxymethylcellulose, and having a tablet hardness of 10 N or more and a disintegration time in water of less than 15 seconds.

2. The orally disintegrating tablet according to Claim 1, further comprising crystalline cellulose.

3. The orally disintegrating tablet according to Claim 1 or 2, further comprising bicarbonate.

4. The orally disintegrating tablet according to any one of Claims 1 to 3, wherein a specific surface of the tablet is of from 0.75 to 1.50 mm²/mg.

5. A method for the production of the orally disintegrating tablet according to any one of Claims 1 to 4, comprising mixing a disintegrative particulate composition with a medicinal ingredient, and subjecting the resulting mixture to tableting.

6. A method for the production of the orally disintegrating tablet according to Claim 5, comprising a wet granulation step in the production of the disintegrative particulate composition.

7. A method for the production of the orally disintegrating tablet according to Claim 5 or 6, comprising a two-stage granulation step in the production of the disintegrative particulate composition.

8. A method for the production of the orally disintegrating tablet according to any one of Claims 5 to 7, wherein the tableting is carried out at a tablet compression force of from 1 to 30 kN.

9. A method for the production of the orally disintegrating tablet according to Claim 8, wherein the tableting is carried out at a tablet compression force of from 2 to 30 kN.

10. A disintegrative particulate composition comprising a first disintegrator component of an acid-type carboxymethylcellulose, for use in the method for the production of the orally disintegrating tablet according to any one of Claims 5 to 9.
